(19) Europäisches Patentamt
European Patent Office
Office européen des brevets

(11) Publication number: **0 172 938**
**A2**

(12) **EUROPEAN PATENT APPLICATION**

(21) Application number: **84113274.9**

(22) Date of filing: **05.11.84**

(51) Int. Cl.⁴: **C 04 B 33/02**

(30) Priority: **06.08.84 HU 1456884**

(43) Date of publication of application: **05.03.86**
**Bulletin 86/10**

(84) Designated Contracting States: **AT BE CH DE FR GB IT LI LU NL SE**

(71) Applicant: **MTA Természettudományi Kutato Laboratoriumai, Budaörsi ut 45, H-112 Budapest (HU)**

(72) Inventor: **Libor, Oszkár, Dr., Dipl.Chem., 4/a, Budenz u., H-1021 Budapest (HU)**
Inventor: **Nagy, Gábor, Dr., Dipl.-Chem., 3, Minerva u., H-1118 Budapest (HU)**
Inventor: **Székely, Tamás, Dr., Dipl.-Chem., 16, Abonyi u., H-1146 Budapest (HU)**

(74) Representative: **Beszédes, Stephan G. Dr., Münchener Strasse 80a Postfach 1168, D-8060 Dachau (DE)**

(54) **A process for preparing clay containing gels.**

(57) The application is concerned with a process for preparing clay mineral containing gels with stabilized structure and reversible water adsorption ability, in which

a) the resistance of medium of aqeous suspension samples of different concentrations containing 1 or more thixotropic, activated, swelling clay mineral(s) of three-layer structure and/or swelling chain silicate(s), optionally containing 1 or more known additive(s), is measured, thereafter in a series of tests there are added to the suspension samples of 1 or more water soluble polymer(s) capable of reacting with the clay mineral(s) and/or chain silicate(s) either as such or in an aqeous solution in different proportions, the resistance of medium of the resulting mixtures is measured, and the clay mineral(s):polymer(s):water ratios are determined at which the resistance of medium of the suspension is at least ten times higher than that of the initial, polymer-free suspension, thus obtaining the useful limits, and then

$b_1$) it is carried out the contacting of the clay mineral(s) and/or chain silicate(s) with the polymer(s) in a ratio falling within the useful limits, in the presence of an amount of water within the useful limits, or

$b_2$) it is carried out the contacting of the clay mineral(s) and/or chain silicate(s) with the polymer(s) in a ratio within or outside the useful limits, in the presence of more water than that corresponding to the useful limits, then the water content of the resulting flocculate is decreased mechanically to fall within the useful limits, and, if necessary, the clay mineral(s):polymer(s) ratio of the mass is adjusted simultaneously to fall within the useful limits by adding further amount(s) of clay mineral(s) and/or chain silicate(s) and/or polymer(s) to the mass and/or optionally 1 or more known additive(s) is or are, respectively, added to it.

Furthermore the application deals with the clay mineral containing gels obtained by this process and their use.

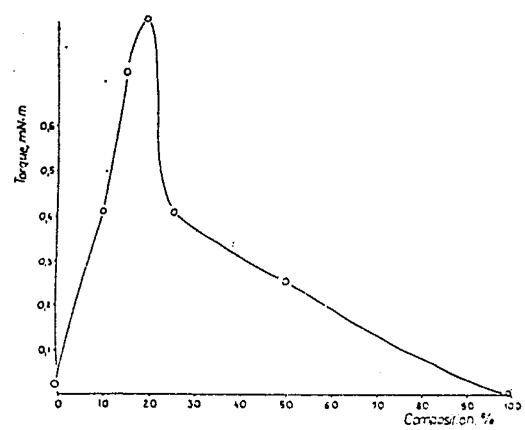


EP 0 172 938 A2

The invention is concerned with a process for preparing clay mineral containing gels with stabilized structure and reversible water adsorption ability by reacting clay minerals with organic polymers as well as with clay mineral containing gels prepared by this process and their use.

It is known that clay minerals react with certain organic polymers by forming various complexes of clay and organic polymers. (B. K. G. Theng: I. The Chemistry of Clay-Organic Reactions, Halsted Press, 1974; II. Polymer-Clay Complexes, Halsted Press, 1979). Such reactions were utilized for various technical purposes. Thus, e.g. it is known that the rheological properties of clay minerals can be modified by admixing them with polymers; this phenomenon is utilized primarily to improve the characteristics of muds (US Patents Nos. 577,588, 693,839 and 250,619; Japanese Patent No. 139,800). It is also known that water soluble polymers can be applied to destabilize or flocculate clay mineral suspensions (US Patents Nos. 3,511,778, 3,701,417, 3,276,998 and 3,052,595; British Patent No. 1,387,744; Japanese Patent No. 081,442). Other publications deal with the utilization of reactions between clay minerals and polymers in the production of retention or coating materials for paper industry (Swiss Patent No. 561,668; British Patent No. 1,010,828). Mixtures or reaction products of clay minerals, organic polymers and other additives can also be applied to produce mortars for underground insulation (US Patent No. 4,043,827), to render soil layers water-proof (US Patent No. 3,772,893), as additives for brick production (US Patent No. 4,148,662) and furthermore for mechanical dehydration and insulation purposes (US Patent No. 442,161).

It is a common characteristic of a part of the processes disclosed in the above publications that polymers are contacted with non-swelling clay minerals or with clay minerals incompletely disintegrated to elementary lamellae or chains. Under such conditions chemical bonds may form between the clay mineral packages and the polymer chains or balls only on the contacting surfaces. The other part of the known processes has the common characteristic that swollen or quickly swelling clay minerals are reacted with a small amount of polymer. In both instances products consisting of inorganic and organic macromolecules are formed, the structure and gelling properties of which are determined primarily by the given characteristics of the inorganic component, the organic component only modifying these properties to a greater or lesser extent. As an example, when a thixotropic, swelling clay mineral of three-layer structure is reacted in non-disintegrated state with an aqueous solution of a known water soluble polymer, the resulting product still remains thixotropic, and the gel formed from the product peptizes like the starting clay mineral; the polymer present modifies only certain other properties, e.g. water adsorption capacity and liquid limit, of the peptizing gel to a greater or lesser extent.

No method has been disclosed so far in the literature which would allow the irreversible fixation (stabilization) of the gel structure of initially thixotropic clay minerals so as to result in a gel having a reversible water uptake/water release ability without any thixotropic character and peptizing ability.

Hence the problem underlying to the invention is to create a process for preparing clay mineral containing gels which by allowing the irreversible fixation (stabilization) of the gel structure of initially thixotropic clay minerals results in gels having a stabilized structure and a reversible water uptake/water release ability without any thixotropic character and peptizing ability as well as the gels prepared according to it and their use.

This surprisingly has been attained by the invention.

Now surprisingly it has been found that when a suspension of appropriate concentration of a thixotropic clay mineral, i.e. a swelling clay mineral, such as smectite, with three--layer structure and/or a swelling chain silicate is contacted with a reactive polymer in activated state, i.e. in a form where the bulk of the clay mineral or chain silicate is disintegrated to its elementary lamellae or chains, the elementary lamellae or chains of the clay mineral are joined to the polymer chains by chemical bonds and/or via adsorption, the lamellae "get stringed" on the polymer, and thus the gel structure of the clay mineral is fixed irreversibly. The resulting gel is no longer thixotropic, i.e. its gel structure cannot be destroyed by mechanical effects, and furthermore, the gel does not peptize and moreover takes up and releases water in a reversible manner.

Hence the subject-matter of the invention is a process for preparing clay mineral containing gels with a stabilized structure and reversible water adsorption ability by contacting aqueous suspensions of 1 or more clay mineral(s), optionally containing 1 or more known additive(s), with 1 or more organic polymer(s) and, optionally, if desired, after the addition of 1 or more known additive(s), to the resulting gel, partially or totally drying it and, if desired, adding to it 1 or more known additive(s), which is characterized in that

a) the resistance of medium of aqueous suspension samples of different concentrations containing 1 or more thixotropic, activated, swelling clay mineral(s) of three-layer structure and/or swelling chain silicate(s) [in the following frequently simply designed as clay mineral(s)], optionally containing 1 or more known additive(s), is

measured, thereafter in a series of tests there are added to the suspension samples samples of 1 or more water soluble polymer(s) capable of reacting with the clay mineral(s) and/or chain silicate(s) either as such or in an aqueous solution in different proportions, the resistance of medium of the resulting mixtures is measured, and the clay mineral(s) : polymer(s) : water ratios are determined at which the resistance of medium of the suspension is at least ten times and at most sixty times higher than that of the initial, polymer-free suspension, thus obtaining the useful limits, and then

$b_1$) it is carried out the contacting of the clay mineral(s) and/or chain silicate(s) with the polymer(s) in a ratio falling within the useful limits, in the presence of an amount of water within the useful limits, or

$b_2$) it is carried out the contacting of the clay mineral(s) and/or chain silicate(s) with the polymer(s) in a ratio within or outside the useful limits, in the presence of more water than that corresponding to the useful limits, then the water content of the resulting flocculate is decreased mechanically to fall within the useful limits (dehydration), and, if necessary, the clay mineral(s) : polymer(s) ratio of the mass is adjusted simultaneously to fall within the useful limits by adding further amounts(s) of clay mineral(s) and/or chain silicate(s) and/or polymer(s) to the mass and/or optionally 1 or more known additive(s) is or are, respectively, added to it,

and optionally it is carried out partial or total drying of the resulting gel, whereafter optionally the partially or totally dried gel is reacted in an aqueous medium with [a] polymer(s) capable of reacting with the clay mineral(s) and/or chain silicate(s) keeping the clay mineral(s) : polymer(s) ratio still within the useful limits and/or 1 or more known additive(s) is or are, respectively, added to it.

Thus by the above group a) of steps of the process according to the invention the clay mineral(s) : polymer(s) : water ratios are determined under which the formation of a gel with the required characteristics takes place.

Preferably for obtaining the useful limits the clay mineral(s) : polymer(s) : water ratios are determined at which the resistance of medium of the suspension is at least twenty times higher than that of the initial, polymer-free suspension. The upper limit of the useful limits of the resistance of medium of the suspension is always the maximum value measurable by determining the maximum torque of the cone of the Brabender type rheometer (cf. Fig.).

According to the above variant $b_1$) of the process according to the invention the gel with the required characteristics is formed directly.

According to the above variant $b_2$) of the process according to the invention the gel with the required characteristics is obtained after the mechanical dehydration step. Suitably the mechanical dehydration is performed by kneading.

Thus in the process according to the invention the required ratios of the clay mineral(s) : polymer(s) : water can be adjusted in one or more steps of contacting.

A single polymer or mixtures of 2 or more polymers can equally be utilized in the process according to the invention. When the gel with the required characteristics is produced in 2 steps, and a polymer is also added to the mass during the mechanical dehydration, the polymers utilized in the first and second steps may be the same or different.

When all the water present is removed in the drying operation optionally carried out, a xerogel powder is obtained.

By the step of reacting the obtained partially or totally dried gel with the polymer(s) the properties of the gel can be modified. Of course, also other known organic and/or mineral additives can be added to the gel in order to modify its final properties.

Of the thixotropic, swelling clay minerals with three--layer structure the montmorillonite-type clay minerals, such as beidellite, hectorite, sepiolite and nontronite, are preferred. These clay minerals can be utilized either alone or in admixture with one another. An advanteously utilizable example of the thixotropic, swelling chain silicates is attapulgite; this clay mineral can be used either alone or in admixture with the three-layer clay minerals listed above. The clay minerals can also be applied in the form of clay mineral containing rocks, e.g. bentonite; according to the experiences of the Applicant the non-clay mineral constituents of the rock do not disturb the reaction, and may sometimes favourably influence the characteristics, e.g. strength or elasticity, of the gel formed. The term "clay mineral" used in the present text also covers such clay mineral containing rocks.

Preferably as an aqueous suspension such containing from 1 to 20% by weight, particularly 3 to 15% by weight, of clay mineral(s) and/or chain silicate(s) [dry substance content] is used.

It is of basic importance that the clay minerals should be utilized in activated state. The term "activated clay mineral" covers swollen clay minerals, which contain mainly $H^+$, $Na^+$, $Li^+$ and/or $NH_4^+$ ions as loosely bound, exchangeable interlamellar cations. The clay minerals or clay mineral containing rocks can have been activated by methods known per se.

The water soluble polymers capable of reacting with clay minerals or in other terms reactive polymers used in the process according to the invention are known per se; of them preferably such containing $-\overset{O}{\overset{\|}{C}}-OH$, $-\overset{O}{\overset{\|}{C}}-O^- M^+$, wherein $M^+$ is a monovalent cation, $-\overset{O}{\overset{\|}{C}}-NH_2$ , $-OH$ and/or $-\overset{|}{\underset{|}{C}}-O-\overset{|}{\underset{|}{C}}-$ groups as functional groups is or are, respectively, used. Examples of reactive polymers which may be used in the process according to the invention are the following: polyacrylamide, polymethacrylamide, polyacrylic acid, polymethacrylic acid, hydrolysed polyacrylamide, acrylamide-acrylic acid copolymers, hydrolysed acrylamide--acrylate copolymers, polyvinyl alcohol-acrylic acid copolymers, polyvinyl alcohols, hydrolysed polyvinyl esters, polyethylene oxides and water soluble polysaccharides as well as mixtures thereof. Also graft copolymers can be applied, examples of which are copolymers containing acrylic acid, methacrylic acid, acrylamide and/or methacrylamide side chains grafted onto a cellulose chain.

Preferably as [a] water soluble polymer(s) such having a molecular weight of 50,000 to 20,000,000, preferably 500,000 to 10,000,000 is or are, respectively, used. According to the experiences of the Applicant the greater the molecular weight of the polymer(s), the smaller amount of polymer(s) is sufficient to stabilize the gel structure of the starting activated clay mineral irreversibly.

It is also of basic importance that the polymer(s) should be reacted with the clay mineral(s) in a sufficiently large amount of water, since it can be ensured only in this case that the polymer balls are sufficiently run off and join to the elementary clay mineral chains or lamellae to fix their structure. It is preferred to add the polymer as an aqueous solution to the aqueous suspension of the clay mineral. Particularly preferably the polymer(s) is or are, respectively, used as an aqueous solution with a viscosity of 50 to 100,000 cP at 20°C.

By the appropriate choice of the clay mineral(s) : polymer(s) weight ratio and the molecular weight of the polymer(s), gels preferably with a viscosity exceeding 100,000 cP at 20°C are prepared. The clay mineral(s) : polymer(s) weight ratios as well as the molecular weight of the polymer(s) required to attain this viscosity can be determined by the test described above.

Various techniques can be applied to contact the clay mineral suspension with the polymer(s) or its or their aqueous solution(s). One may e.g. simply admix the suspension with the polymer(s) or its or their aqueous solution(s). However, it is more preferred to admix the clay mineral suspension with the aqueous solution(s) of the polymer(s) in a spray head and then spraying the mixture. Alternatively, the aqueous solution of the polymer(s) and the aqueous suspension of the clay mineral(s) may be sprayed from 2 or more separate spray heads, whereupon the droplets contact one another in the air. Using this latter method, the gel structure is stabilized simultaneously with the partial or total drying of the gel.

As mentioned above, if desired, various additives can be added to the gel in order to modify the properties of the end product. As such suitably 1 or more filling agent(s) and/or modifying agent(s), e.g. natural or synthetic fibrous material(s), inert or activated particulate filler(s), such as quartz sand and/or ground basalt, and/or hydraulic binding agent(s), such as alkaline fly ash, and/or furthermore grass seeds and/or nutrient substance(s) can be used. These filling and modifying agents are known per se. Depending on their chemical nature, the filling and/or modifying agent(s) can be added to the clay mineral suspension, i.e. to the gel with non-stabilized structure, to the wet, stabilized gel and/or to the partially or totally dried stabilized gel.

A subject-matter of the invention are also clay mineral containing gels which are characterized in that they have been prepared by the process according to the invention.

A major advantage of the process according to the invention is that a stable gel structure can be formed even when reacting the clay mineral(s) with a relatively small amount of polymer(s). Upon stabilizing the gel structure, the utilization fields of the starting clay mineral can be broadened to a great extent.

Thus, e.g. the gels prepared by the process according to the invention can be utilized as water insulating materials, particularly in the building industry, primarily as insulators for structural engineering, as rock strengthening and water insulating agents in the mining industry, for the environment--proof insulation and closing of waste containers, in the agriculture, primarily to increase the water retention ability of loose sandy soils, as soil stabilizing agents and for fire extinguishing.

Hence furthermore a subject-matter of the invention is the use of the clay mineral containing gels according to the invention as environment-proof insulating or jointing, particularly water insulating, materials, rock strengthening agents, agents for increasing the water retention ability, particularly of soils, soil stabilizing agents or for fire extinguishing.

The use of the clay mineral containing gels according to the invention can be effected also in the way that they are prepared by the process according to the invention in situ at the site of application, for example on surfaces at the construction site.

The invention is elucidated in detail by the aid of the following Examples.

## Example 1

A) An aqueous suspension of 10% by weight dry substance content is prepared from commercially available activated bentonite (B1 type bentonite, produced by Erbslöh Co., Germany) under laboratory conditions. The suspension is sampled, and the samples are filled into a Brabender type rheometer. The torque of the cone which rotates in the rheometer is measured at 20°C; the measured torque is characteristic of the resistance of medium. Then, varying amounts of polyacrylamide (average molecular weight: 3,000,000) are added, as an 5% aqueous solution, to the suspension samples, the suspensions are admixed with the solution, and the torques of the mixtures are measured again. The measured torques are plotted against the weight ratio of the polymer in the mixture (see Fig.). It can be seen from the

0172938

curve that for the polyacrylamide used and with the water content applied in the test, a clay mineral : polymer weight ratio of 95 : 5 to 43 : 57 should be maintained in order to obtain a non-thixotropic, non-peptizing gel with stabilized structure, which has reversible water adsorption ability.

In the following the ratios determined as described above are maintained when preparing a gel.

B) 1,000 g of an aqueous suspension, containing 10% by weight of activated bentonite defined under section A), are prepared under laboratory conditions. In the following, 50 g amounts each of the thixotropic substance, swollen to equilibrium, are reacted individually with the polymers listed in the following Table 1.

Table 1

| No. of solution | Polymer | Average molecular weight | Concentration of the solution in % by weight |
|---|---|---|---|
| B1 | Polyacrylamide | 10,000,000 | 0.2 |
| B2 | Polyacrylamide | 3,000,000 | 0.5 |
| B3 | Polyacrylamide | 300,000 | 2 |
| B4 | Hydrolysed polyacrylamide (hydrolysis degree: 3%) | 800,000 | 2 |

- 13 -

Table 1

(Continued)

| No. of solution | Polymer | Average molecular weight | Concentration of the solution in % by weight |
|---|---|---|---|
| B5 | Hydrolysed polyacrylamide (hydrolysis degree: 40%) | 10,000,000 | 0.1 |
| B6 | Hydrolysed polymethacrylamide (hydrolysis degree: 10%) | 1,000,000 | 0.8 |
| B7 | Acrylamide-acrylic acid copolymer (acrylamide content: 20% by weight) | 2,000,000 | 0.5 |
| B8 | Acrylamide-maleic anhydride copolymer [alternating] (acrylamide content: 43% by weight) | 500,000 | 3 |
| B9 | Hydrolysed vinyl acetate-acrylic acid copolymer (acrylic acid content: 20% by weight) | 700,000 | 2 |

- 14 -

Table 1

(Continued)

| No. of solution | Polymer | Average molecular weight | Concentration of the solution in % by weight |
|---|---|---|---|
| B10 | Hydrolysed methyl-methacrylate-methacrylic acid copolymer (containing maximum 10% of non-hydrolysed ester groups | 500,000 | 4 |

The polymers are utilized as aqueous solutions. Each of the polymer solutions has a viscosity exceeding 500 cP at 20°C. 20 ml each of the polymer solutions are admixed individually with 50 g each of the bentonite gel in a beaker of 100 ml capacity. After 10 to 60 seconds the viscosity of the mixture increases abruptly, and a stable gel is formed. The strength of the gel is such that it can be removed from the beaker in a single piece and does not break under its own weight.

All the resulting gels are water-insoluble, they take up and release water reversibly, and do not show syneresis.

The limiting values of the clay mineral : polymer weight ratios were determined according to the test

described under section A); the weight ratios used under section B) of this example are within the limiting values.

Example 2

One proceeds as described in Example 1, section B) with the difference that a homogeneous mixture of 10 ml each of solutions B1 and B9 is added to the bentonite suspension. The reaction of the 2 polymers with the clay mineral takes practically the same time, and an apparently homogeneous gel is formed within about 1 minute. The resulting gel is, however, heterogeneous, i.e. the clay mineral lamellae are fixed by 2 polymeric matrices different in chemical structure. The properties of the gel are the same as given in Example 1.

Example 3

A 6% by weight aqueous suspension is prepared from commercially available activated bentonite of type F1 (produced at Mád, Hungary). Aliquots of the suspension are admixed individually with the filling agents listed in the following Table 2.

Table 2

| No. of filler | Filler | Amount of filler in % by weight calculated for the suspension |
|---|---|---|
| A1 | Quartz powder (type 00) | 10 |
| A2 | Asbestos wool (fibre length: 0.5 to 1.5 mm) | 2 |
| A3 | Dry sodium cellulose | 3 |
| A4 | Cement powder (admixable with bentonites) | 4 |

The resulting suspensions are admixed with polymer solution B5 of Example 1 in a weight ratio of 5 : 2. The compression strength and resistance to crack of the resulting gels have been improved significantly by the effect of the filling agents; the compression strength of the resulting gels is 2- to 3-fold of that of the non-filled one. These filler containing gels can be applied primarily for the production of water-insulating screens.

Example 4

The bentonite suspension defined in Example 1, section A) and the polymer solution B4 defined in Example 1, section B) are introduced simultaneously in a volume ratio of 5 : 2, through a mixing tube, into the upper part of a filled geyser

dryer, at an inlet 10 cm below the glass bead filling. The filling is moved in the centre by a pulley stirrer, and is kept floating by a hot (120°C) air stream introduced at the bottom of the dryer. The liquid substance, entering through the mixing tube, is deposited on the filling and gels simultaneously, whereafter the thin gel layer dries, is comminuted, and a fine xerogel powder separates in the cyclon part. The resulting fine powder, with an average particle size of about 50 µm, is swelling, and has a reversible water adsorption ability. The water tightness of the resulting gel is far higher than that of the initial bentonite (the K value of the initial bentonite is in the order of $10^{-8}$, whereas that of the gel only about $10^{-10}$). The drying temperature should be so controlled that the average temperature of the particles does not exceed 90°C.

## Example 5

The swollen bentonite suspension of the composition indicated in Example 3 and the aqueous polymer solution B1 defined in Example 1, section B) are introducted simultaneously, in a volume ratio of 4 : 1, into an Anhydro type disc spray dryer. The sprayed droplets gel, and a fine xerogel powder, with an average particle size of 20 µm, separates in the cyclon. The properties of the resulting product are very close to those of the xerogel obtained according to Example 4. The drying temperature should be so controlled that the average temperature of the particles does not exceed 90°C.

The obtained xerogel powder can be subjected to the following further treatments.

I) 8 g of the xerogel powder prepared as indicated in the foregoing are suspended in 92 ml of distilled water, and 20 ml of a polymer solution B1, defined in Example 1, section B), are added to the suspension with stirring. After about 30 seconds of stirring a stable gel is obtained, with an elongation at rupture of about 200%.

II) One proceeds as described in section I) with the difference that an 0.5% aqueous solution of a polyethylene oxide (Polyox® Coagulant Covale; average molecular weight: about 6,000,000) is added to the suspension. After about 40 seconds of stirring a gel is formed, followed by a syneresis stage of about 10% after further 10 minutes. The mechanical strength of the resulting gel is particularly favourable; its elongation at rupture is about 200%, and its strength at rupture is about the double of that of the product obtained according to section I).

## Example 6

The bentonite suspension and the polymer solution described in Example 5 are sprayed simultaneously, from 2 separate tanks equipped with spray devices, onto the surface of freshly built storm banks subject to heavy soil erosion. Where the droplets form a coherent layer, they are partially adsorbed by the upper surface of the soil, and the resulting gel sticks the soil particles together and stabilizes them. The gel layer does not interfere with plant growth. It is also possible to introduce grass seeds and/or nutrient substances into the polymer solution, to bring them onto the treated surface simultaneously with gel formation.

Example 7

0.8 mg of a partially hydrolysed polyacrylamide (degree of hydrolysis: 20%, average molecular weight: above $10^7$) is added, as a 0.1% aqueous solution, to a suspension of 400 mg of technical kaolinite, 50 mg of quartz powder (particle size: below 10 µm) and 650 mg of activated bentonite (type F1, produced at Mád, Hungary) in 1 litre of water. The suspension is stirred for 2 minutes at a rate of 200 r.p.m., then for 5 minutes at a rate of 20 r.p.m. The resulting flocculate sediments in 15 minutes. The water above the sediment layer is removed, the sediment, 80 ml in volume, is stirred at a rate of 10 r.p.m., and 2% by weight, calculated on the weight of the bentonite, of the above polymer are added to the sediment as an 0.5% aqueous solution, whereupon the volume of the sediment decreases to 20 ml. 60 ml of water above the sediment are removed, and the sediment is kneaded in the kneading chamber of a Brabender plastograph at a rate of 60 r.p.m. After 10 minutes of kneading a homogeneous gel is formed from the sediment, with a simultaneous loss of water.

Claims

## Claims

1.) A process for preparing clay mineral containing gels with stabilized structure and reversible water adsorption ability by contacting aqueous suspensions of 1 or more clay mineral(s), optionally containing 1 or more known additive(s), with 1 or more organic polymer(s) and, optionally, if desired, after the addition of 1 or more known additive(s), to the resulting gel, partially or totally drying it and, if desired, adding to it 1 or more known additive(s), characterized in that

a) the resistance of medium of aqueous suspension samples of different concentrations containing 1 or more thixotropic, activated, swelling clay mineral(s) of three-layer structure and/or swelling chain silicate(s), optionally containing 1 or more known additive(s), is measured, thereafter in a series of tests there are added to the suspension samples samples of 1 or more water soluble polymer(s) capable of reacting with the clay mineral(s) and/or chain silicate(s) either as such or in an aqueous solution in different proportions, the resistance of medium of the resulting mixtures is measured, and the clay mineral(s) : polymer(s) : water ratios are determined at which the resistance of medium of the suspension is at least ten times and at most sixty times higher than that of the initial, polymer-free suspension, thus obtaining the useful limits, and then

$b_1$) it is carried out the contacting of the clay mineral(s) and/or chain silicate(s) with

the polymer(s) in a ratio falling within the useful limits, in the presence of an amount of water within the useful limits, or

$b_2$) it is carried out the contacting of the clay mineral(s) and/or chain silicate(s) with the polymer(s) in a ratio within or outside the useful limits, in the presence of more water than that corresponding to the useful limits, then the water content of the resulting flocculate is decreased mechanically to fall within the useful limits, and, if necessary, the clay mineral(s) : polymer(s) ratio of the mass is adjusted simultaneously to fall within the useful limits by adding further amounts(s) of clay mineral(s) and/or chain silicate(s) and/or polymer(s) to the mass and/or optionally 1 or more known additive(s) is or are, respectively, added to it,

and optionally it is carried out partial or total drying of the resulting gel, whereafter optionally the partially or totally dried gel is reacted in an aqueous medium with [a] polymer(s) capable of reacting with the clay mineral(s) and/or chain silicate(s) keeping the clay mineral(s) : polymer(s) ratio still within the useful limits and/or 1 or more known additive(s) is or are, respectively, added to it.

2.) A process as claimed in claim 1, characterized in that for obtaining the useful limits the clay mineral(s) : polymer(s) : water ratios are determined at which the resistance of medium of the suspension is at least twenty times higher than that of the initial, polymer-free suspension.

3.) A process as claimed in claim 1 or 2, characterized in that as an aqueous suspension such containing from 1 to 20% by weight, particularly 3 to 15% by weight, of clay mineral(s) and/or chain silicate(s) is used.

4.) A process as claimed in claims 1 to 3, characterized in that as [a] water soluble polymer(s) such containing $-\overset{O}{\overset{\|}{C}}-OH$, $-\overset{O}{\overset{\|}{C}}-O^{-}M^{+}$, wherein $M^{+}$ is a monovalent cation, $-\overset{O}{\overset{\|}{C}}-NH_2$, $-OH$ and/or $-\overset{|}{\underset{|}{C}}-O-\overset{|}{\underset{|}{C}}-$ groups as functional groups is or are, respectively, used.

5.) A process as claimed in claims 1 to 4, characterized in that as [a] water soluble polymer(s) such having a molecular weight of 50,000 to 20,000,000, preferably 500,000 to 10,000,000 is or are, respectively, used.

6.) A process as claimed in claims 1 to 5, characterized in that the polymer(s) is or are, respectively, used as an aqueous solution with a viscosity of 50 to 100,000 cP at 20°C.

7.) Clay mineral containing gels with stabilized structure and reversible water adsorption ability, characterized in that they have been prepared by the process according to any of claims 1 to 6.

8.) The use of the clay mineral containing gels according to claim 7 as environment-proof insulating or jointing, particularly water insulating, materials, rock strengthening agents, agents for increasing the water retention ability, particularly of soils, soil stabilizing agents or for fire extinguishing.

1/1

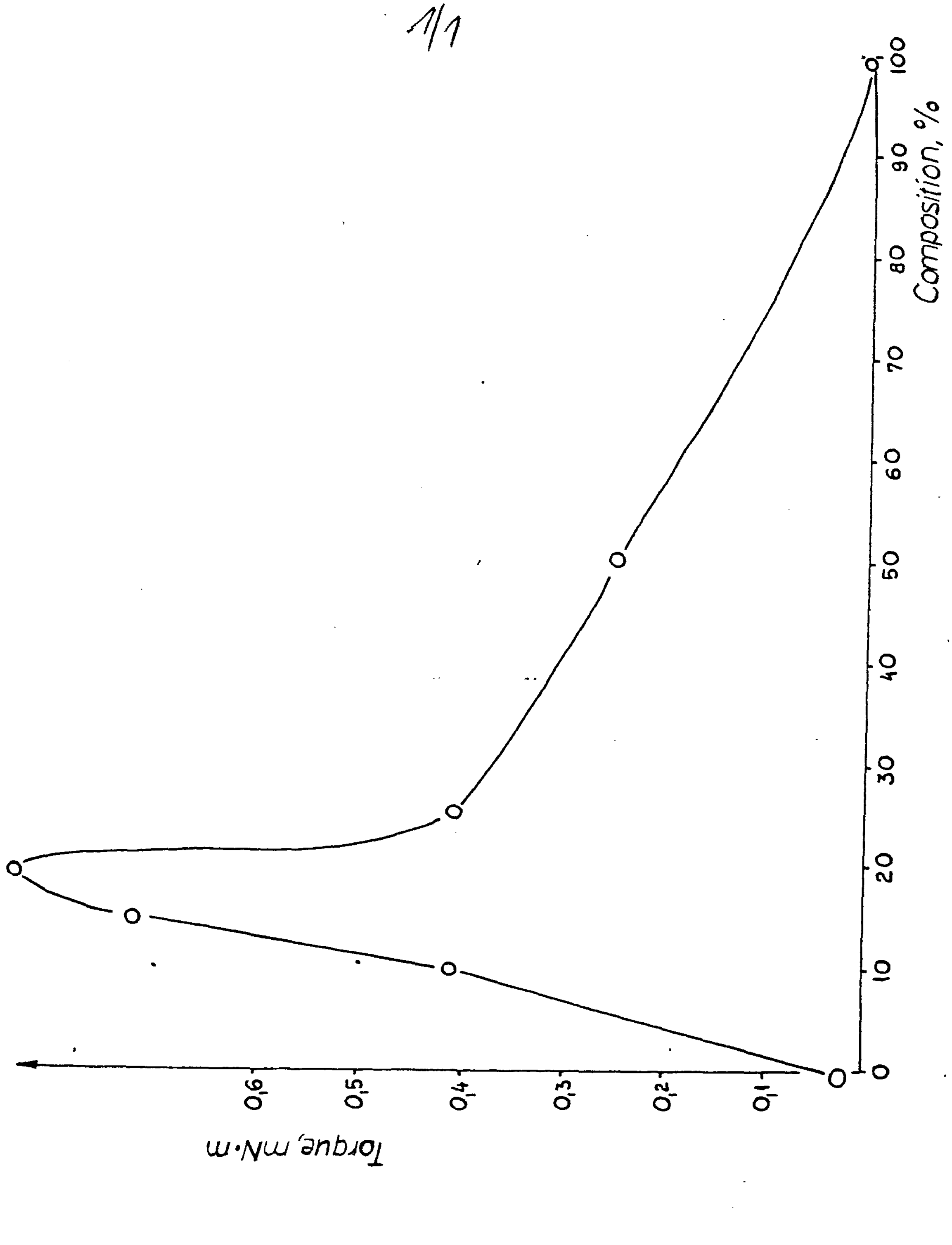
Torque, mN·m
0,1
0,2
0,3
0,4
0,5
0,6
Composition, %
0
10
20
30
40
50
60
70
80
90
100